# EUROPÄISCHE PATENTANMELDUNG

(11) **EP 3 660 860 A1**
(43) Veröffentlichungstag der Anmeldung: **03.06.2020**
(21) Anmeldenummer: 18208622.3
(22) Anmeldetag: 27.11.2018
(51) Int. Cl.: G16H 40/63, G16H 40/67

(54) **VERFAHREN UND VORRICHTUNG ZUR STEUERUNG EINER ANZEIGEEINHEIT IN EINEM MEDIZINTECHNISCHEN GERÄTESYSTEM**

(71) Anmelder: Siemens Healthcare GmbH, 91052 Erlangen (DE)
(72) Erfinder: Hofmann, Bernd, 91058 Erlangen (DE); Kaup, Ina, 91052 Erlangen (DE); Ullrich, Christian, 90482 Nürnberg (DE)

(57) **Zusammenfassung**

Die Erfindung betrifft ein Verfahren zur Kommunikation mit einem medizintechnischen Gerätesystem (GS) durch einen Benutzer (2) während einer Operation in einem Operationssaal, umfassend die Schritte:
- Bereitstellen von Zustandsdaten (Z) über eine aktuelle Situation im Operationssaal und/oder von Handlungen des Benutzers (2),
- Analyse der Zustandsdaten (Z) mittels eines trainierten Algorithmus (A) basierend auf einer künstlichen Intelligenz,
- datentechnische Kommunikation mit Geräten (G1, G2, G3) des Gerätesystems (GS) basierend auf der Analyse, wobei diese Kommunikation
a) eine Steuerung und/oder
b) ein Abrufen von Daten (D)
dieser Geräte (G1, G2, G3) umfasst.

Die Erfindung betrifft darüber hinaus eine diesbezügliche Vorrichtung sowie ein medizintechnisches Gerätesystem.

## Beschreibung

Die Erfindung betrifft ein Verfahren und eine Vorrichtung zur Steuerung einer Anzeigeeinheit in einem medizintechnischen System sowie ein medizintechnisches Gerätesystem mit einer solchen Vorrichtung.

Fortschritte in der Medizin führen dazu, dass im modernen Operations-Umfeld die unterschiedlichsten Technologien zum Einsatz kommen. Diese Technologien sind in der Regel herstellerabhängig und an ein individuelles Medizingerät gebunden. In der Regel hat jedes Medizingerät seine eigne Ein- und Ausgabeinfrastuktur. Dies führt dazu, dass bei einer Operation im ohnehin schon räumlich begrenzten Umfeld des Operateurs oftmals eine Vielzahl von Ein- und Ausgabegeräten platziert sind. Diese Geräte sind in der Regel in einer für den Operateur nicht optimalen Weise platziert, sondern an Stellen, an denen sie den OP-Verlauf am wenigsten stören, was der räumlichen Enge geschuldet ist. Dies hat jedoch den Nachteil dass die verschiedenen, für den Operateur wichtigen Informationen, welche die Medizingeräte liefern an unterschiedlichen Stellen im Raum verteilt sind und der Operateur zum Abruf dieser Informationen ständig seine Blickrichtung vom Patienten zu den unterschiedlichen Geräten abwenden muss.

In der Praxis muss also der Operateur die Informationsquelle zunächst im Raum lokalisieren, diese Informationen memorieren und sich schließlich zurück in seine Arbeitsposition begeben und sich erneut dem Fokus seiner Tätigkeit zuwenden. Dies hat den Nachteil dass der Operateur durch die dadurch bedingte unergonomische Körperhaltungen und -bewegungen, permanentes optisches (De)-fokussieren auf verschiedene "Points of Interest" und einen vermehrten kognitiven Erinnerungsaufwand rasch ermüdet, was das Risiko von Fehlern erhöht. Gleichzeitig führt der ständige Wechsel des Aufmerksamkeitsfokus und der Arbeitshaltung zu einem unnötigen Zeitverlust, der die Operationsdauer verlängert und damit die Risiken der Narkoseführung erhöht.

Um diesen Zeitverlust auszugleichen, wägt der Operateur ständig ab, ob die wiederholte Einholung der Information notwendig ist oder ob er alternativ auf erinnerte Informationen zurückgreift. Diese erinnerten Informationen können fehlerhaft oder veraltet sein und zu falschen Schlussfolgerungen führen. Verbunden mit einem Anstieg des Stresslevels des Operateurs in unerwarteten oder schwierigen Situationen, wie sie bei einer Operation durchaus auftreten, wird die oben beschriebene Problematik noch verstärkt.

Zusätzlich zu den oben genannten Nachteilen wird die Arbeit eines Operateurs noch durch weitere typische Aspekte erschwert, die im Folgenden aufgezählt sind.
a) Die räumliche Enge des OP-Saals führt häufig dazu, dass der Blick des Operateurs auf die wesentlichen Informationen durch andere Gegenstände oder Personen verdeckt wird. Abhilfe kann der Operateur nur dadurch schaffen, dass er im Saal anwesendes Personal auffordert dies zu ändern. Dies führt dazu, dass relevante Informationen nur verzögert abgerufen werden können.
b) Aufgrund von unterschiedlicher Größe und Konzeption bzw. Grundriss der Operationssäle, sowie dem variierenden Bedarf an Geräten und unterschiedlicher Präferenzen der Akteure, ist die räumliche Positionierung der Informationsquellen nur wenig standardisiert. Dies führt dazu, dass sich der Operateur bei jeder Operation auf die jeweilige räumliche Situation neu einstellen muss. Dies führt zu einer Abnahme der Fähigkeit für fertigkeitsbasiertes Arbeiten.
c) Häufig ist es notwendig, dass der Operateur auf verschiedene Informationen, die von einem Gerät bzw. einer Technologie zur Verfügung gestellt werden, zurückgreift. Hierzu ist z.B. bei der Schnittbilddiagnostik ein zeitaufwändiges Scrollen durch die einzelnen Bilder notwendig. Dies kann entweder durch den Operateur mithilfe steriler Eingabegeräte oder auf Anweisung durch weiteres OP-Personal erfolgen. Beides führt zu einer Verzögerung des OP-Fortschrittes und zu einer Defokussierung des Operateurs. Derzeit ist es nicht möglich die entsprechend dem OP-Fortschritt benötigten Informationen spezifisch anzuzeigen. Die gezielte Informations-Extraktion wird dadurch erschwert.
d) Eine Bereitstellung der jeweils notwendigen Informationen entsprechend dem OP-Fortschritt im Sinne eines Ablaufplanes findet derzeit nicht statt. Wären solche Informationen vorhanden, könnte sich der Operateur auf seine Tätigkeit konzentrieren und es würde sichergestellt werden, dass alle wesentlichen Schritte einer Operation auch durchgeführt werden.

Die Steuerung der verschiedenen Geräte wird bisher auf Anweisung des Operateurs von Hilfspersonal im OP durchgeführt (z.B. eine Änderung der Stärke der Gewebekoagulation an unterschiedlichen Strukturen). Dies beinhaltet zum einen eine Zeitverzögerung, zum anderen können aufgrund von Kommunikationsschwierigkeiten Eingabefehler erfolgen. Dies kann zu Fehlfunktionen und ungewollten Verletzungen des Patienten führen.

Abhilfe war bisher, dass die Einstellungen vom Operateur visuell kontrolliert wurden oder eine verbale Bestätigung der Einstellung durch das OP-Personal erfolgte.

Die Gesamtheit dieser Aspekte führt zu einer vermehrten körperlichen und kognitiven Belastung sowie einem Nachlassen der Zuverlässigkeit im Handeln des Operateurs, welcher unmittelbar mit einer erhöhten Gefährdung des Patienten durch potentiell auftretende Operationskomplikationen oder Fehlbehandlungen einhergeht. Dies führt zu folgenden negativen Auswirkungen auf verschiedenen Ebenen:
- Aufgrund der höheren Fehleranfälligkeit im Handeln des Operateurs kann ein schlechteres oder suboptimales klinisches Ergebnis die Folge sein. Dies hat unmittelbare Konsequenzen für die Lebensqualität des Patienten. Weiterhin führt dies zu erhöhten Folgekosten für das Sozial- und Gesundheitssystem.
- Eine Verlängerung der Operationszeit oder eine komplikationsbedingte Verlängerung der Liegezeit führen zu erhöhten Behandlungskosten des betreffenden Krankenhauses.
- Für den Operateur selbst, sowie das gesamte Operationspersonal ergibt sich ein unattraktiver Arbeitsplatz verbunden mit einer erhöhten gesundheitlichen Belastung und einer Zunahme berufsbedingter Erkrankungen (Rückenleiden, Sehstören, Kopfschmerzen). Dies verstärkt unter anderem den derzeit bestehenden Facharztmangel in chirurgischen Disziplinen und verursacht zusätzliche Kosten im Gesundheitswesen.

Die bisherige Problemlösung basierte auf dem Versuch einer standardisierten Geräte-Positionierung im OP-Saal. Da die eingesetzten Geräte jedoch abhängig von der durchgeführten Prozedur sind, kann diese Standardisierung nur auf Basis des jeweiligen Eingriffs erfolgen. Dies hat zur Folge, dass ein Gerät bei verschiedenen Eingriffen entsprechend seiner jeweiligen Wichtigkeit an unterschiedlichen Stellen positioniert wird und daher keine stringente, alle Eingriffe umfassende Standardisierung erfolgen kann. In einem weiteren Schritt wurde, insbesondere herstellerbezogen, die Informationen mehrerer Geräte auf einem Monitor zusammengefasst. Eine Standardisierung aller im OP-Saal vorhandenen Geräte ist jedoch nicht erfolgt.

Ein wesentlicher Nachteil bleibt auch bei diesem Verfahren bestehen: Der Operateur muss seinen visuellen Fokus weiterhin zwischen dem OP-Situs und dem Monitor bewegen. Die oben aufgeführten negativen Auswirkungen bleiben somit größtenteils weiterhin bestehen. Die technische Weiterentwicklung lässt erwarten, dass in Zukunft noch mehr in Geräte in den OP-Ablauf integriert werden und die Situation somit verschärfen.

Es ist eine Aufgabe der vorliegenden Erfindung, ein alternatives, komfortableres Verfahren und eine entsprechende Vorrichtung zur Steuerung einer Anzeigeeinheit in einem medizintechnischen Gerätesystem anzugeben, mit dem die oben beschriebenen Nachteile vermieden werden.

Diese Aufgabe wird durch ein Verfahren gemäß Patentanspruch 1, eine Vorrichtung gemäß Patentanspruch 10, sowie ein medizintechnisches Gerätesystem gemäß Patentanspruch 13 gelöst.

Das erfindungsgemäße Verfahren dient zur Kommunikation mit einem medizintechnischen Gerätesystem, insbesondere zur Steuerung einer Anzeigeeinheit oder von Geräten in diesem Gerätesystem. Sie ist anwendbar für einen Benutzer während einer Operation in einem Operationssaal.

Das erfindungsgemäße Verfahren umfasst die folgenden Schritte:
- Bereitstellen von Zustandsdaten über eine aktuelle Situation im Operationssaal. Typischerweise ist damit eine Situation während einer Operation gemeint, also ein Zustand von Geräten, Messwerten und Personen mit ihren Handlungen in dem Operationssaal. Eine Operation folgt in der Regel einem bestimmten Workflow der sich ggf. situationsbedingt (z.B. in einem Notfall) zwar auch ändern kann, jedoch dabei lediglich durch einen anderen Workflow abgelöst wird. Der Zustand der Patienten und der Geräte sowie die Handlungen der Personen im Operationssaal ergeben die aktuelle Situation. Beispielsweise kann die Bereitstellung von gewissen medizinischen Werkzeugen und einer bestimmten Aufstellung von Personen relativ zum Patienten andeuten, dass nun die Operation beginnt. In diesem Falle wäre die aktuelle Situation: Beginn der Operation. Es kann aber auch ein Benutzer des Verfahrens einen Befehl geben, z.B. "Stelle mir die neuesten Röntgenbilder dar!", dann wäre die aktuelle Situation "Anzeige von Röntgenbildern wird gewünscht".

Die Zustandsdaten sind bevorzugt (aktuelle) Videodaten und/oder Audiodaten aus dem Operationssaal, können aber bevorzugt auch Zustände von Geräten und Messwerte umfassen. Dies bedeutet, dass die Zustandsdaten Aufnahmen einer Kamera und/oder eines Mikrofons umfassen können. Alternativ oder zusätzlich können die Zustandsdaten auch Geräteausgaben umfassen, (z.B. von einem Mikroskop oder einem anderen Bildgebenden Gerät, z.B. mit einer Erfassung von komplexen anatomischen Informationen).
- Analyse der Zustandsdaten mittels eines trainierten Algorithmus basierend auf einer künstlichen Intelligenz. Die bereitgestellten Zustandsdaten sind zunächst einer automatisierten Recheneinrichtung nicht verständlich. Somit müssen diese Zustandsdaten zunächst analysiert werden. Diese Analyse wird durch einen trainierten Algorithmus basierend auf einer künstlichen Intelligenz durchgeführt. Da sich wie oben gesagt, typische Abläufe während einer Operation in typische Workflows einordnen lassen, lässt sich die aktuelle Situation aus den Zustandsdaten ermitteln. Ein Training kann also mit den entsprechenden Zustandsdaten (z.B. Video- und Audiodaten) anderer Operationen durchgeführt werden. Mittels einer Objekterkennung kann z.B. die Position von Personen für eine Recheneinheit verständlich gemacht werden und aus der Position der Personen, ggf. zusammen mit typischen akustischen Anweisungen des operierenden Arztes (und einer in den Trainingsdaten vorgegebenen Art der Situation) ein effektives Training durchgeführt werden.

Die künstliche Intelligenz hat also wiederkehrende Handlungsmuster erlernt, und zieht daraus Rückschluss auf den Workflow. Es kann also die aktuell vorliegende Situation, typischerweise der aktuelle oder anliegende OP-Schritt, mittels der Analyse ermittelt werden.

Eine grafische Analyse kann in der Praxis z.B. mittels einer Standard-Bildanalyse und -Klassifikation basierend auf eindeutigen Merkmalen erreicht werden (z.B. Positionen von Personen im OP), die je nach Algorithmus verschieden sein können. Eine akustische Analyse kann in der Praxis z.B. mittels einer Ableitung von Mel-Cepstrum-Koeffizienten aus der Spektralanalyse der Stimme oder einer Bestimmung von Wort- und Satzketten aus Hidden-Markov Modellen oder mittels eines neuronalen Netzes ermittelt werden.

Es kann auch eine 3D Umgebungsanalyse mittels Erkennungsmodellen basierend auf 3D-Gittern erfolgen, welche auf Grund der Einheitlichkeit echter 3D Modelle trainiert werden. Beispielsweise sind 2 Skalpelle unterschiedlicher Form dennoch als Skalpell klassifizierbar.

Die aktuelle Situation kann somit computerverständlich analysiert werden, z.B. mit einer individuellen Nummer charakterisiert werden, unter der ein standardgemäßes Handlungsprotokoll für den folgenden Schritt in einer Datenbank abgerufen werden kann. Dieser Algorithmus kann aber auch so ausgebildet sein, dass er direkt Handlungsvorgaben für den folgenden Schritt ausgibt.
- Datentechnische Kommunikation mit Geräten des Gerätesystems basierend auf der Analyse. Diese Kommunikation umfasst dabei eine Steuerung und/oder ein Abrufen von Daten dieser Geräte. Dabei sei klargestellt, dass mit dieser Kommunikation nicht die vorangehende Übermittlung von Zustandsdaten gemeint ist, sondern eine Kommunikation, die erst nach der Analyse erfolgt. Die hier abgerufenen Daten dienen bevorzugt zur Anzeige für den Benutzer und werden basierend auf der Analyse ausgewählt.

Es können also beispielsweise gezielt Daten von Geräten abgerufen werden, z.B. die oben erwähnten Röntgenbilder, wenn es die Situation erfordert. Es kann auch eine Veränderung von Geräteeinstellungen erfolgen, z.B. eine Änderung der Koagulationsstärke). Aufgrund der komplexen Verzahnung des Arbeitsplatzes im Operationsfeld, wird bevorzugt die gesamte Infrastruktur des OP-Saales, z.B. Beleuchtung, Klima oder Musik, in die datentechnische Kommunikation, insbesondere die Steuerung, mit einbezogen.

Mit diesem Verfahren kann also eine künstliche Intelligenz zur Kombination verschiedener Informationsquellen und kontextabhängiger Bereitstellung der Informationen bzw. Reaktion auf einen konkreten Operationsablauf erfolgen sowie eine automatisierte Steuerung von Geräten vorgenommen werden.

Die einzelnen Geräte, die bevorzugt Messgeräte (z.B. bildgebende Geräte, Geräte zur Messung von Patientenparametern oder Anzeigegeräte), automatisierte Operationswerkzeuge (z.B. Laser) oder Geräte zur Regelung der Umgebungsparameter (z.B. Licht, Temperatur, Luft) sind, sind bevorzugt in einem drahtgebundenen und/oder drahtlosen Netzwerk (z.B. einem Hochleistungs-WLAN mit z.B. 5GHz, 1Gbit/sec Übertragungsrate) eingebunden. Die Geräte können dann ein gemeinsames real-time Protokoll für den Austausch von Zustandsdaten implementieren. Der Transport der Bilddaten erfolgt bevorzugt mit Methoden der Inter-Prozess-Kommunikation z.B. über ein Software-Interface. Beispielsweise können bestehende medizinische Systeme auf einfache Weise über eine gemeinsame Schnittstelle wie z.B. FHIR (Fast Healthcare Interoperable Resources) in ein Gerätesystem integriert werden.

Die erfindungsgemäße Vorrichtung dient wie das Verfahren zur Kommunikation mit einem medizintechnischen Gerätesystem, insbesondere zur Steuerung einer Anzeigeeinheit oder von Geräten in diesem Gerätesystem. Sie kann durch einen Benutzer während einer Operation in einem Operationssaal verwendet werden. Die erfindungsgemäße Vorrichtung umfasst die folgenden Komponenten:
- Eine Datenschnittstelle zum Empfang von Zustandsdaten über eine aktuelle Situation im Operationssaal und/oder von Handlungen des Benutzers, typischerweise während einer Operation.
- Eine Recheneinheit mit einem trainierten Algorithmus basierend auf einer künstlichen Intelligenz ausgelegt zur Analyse der Zustandsdaten.
- Eine Datenschnittstelle ausgelegt zur datentechnischen Kommunikation mit Geräten des Gerätesystems basierend auf der Analyse. Diese Kommunikation umfasst dabei eine Steuerung dieser Geräte und/oder ein Abrufen von Daten von diesen Geräten.

Ein erfindungsgemäßes medizintechnisches Gerätesystem umfasst eine erfindungsgemäße Vorrichtung und/oder ist zur Durchführung eines erfindungsgemäßen Verfahrens ausgelegt.

Ein Großteil der zuvor genannten Komponenten der Vorrichtung können ganz oder teilweise in Form von Software- oder Hardwaremodulen in einem Prozessor eines Rechensystems realisiert werden. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Rechensystems ladbar ist, mit Programmabschnitten, um alle Schritte des erfindungsgemäßen Verfahrens auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem und/oder zur Speicherung an oder in dem Rechensystem kann ein computerlesbares Medium, beispielsweise ein Memorystick, eine Festplatte oder ein sonstiger transportabler oder fest eingebauter Datenträger dienen, auf welchem die einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung. Dabei kann die erfindungsgemäße Vorrichtung bzw. das erfindungsgemäße medizintechnische Gerätesystem auch analog zu den abhängigen Verfahrensansprüchen oder Beschreibungsteilen weitergebildet sein und insbesondere auch einzelne Merkmale verschiedener Ausführungsbeispiele bzw. Varianten zu neuen Ausführungsbeispielen bzw. Varianten kombiniert werden können.

Ein bevorzugtes Verfahren umfasst die folgenden Schritte:
- Ermittlung derjenigen Menge von Daten ("Datenmenge") welche während der aktuellen Situation im Operationssaal auf einem Anzeigegerät ausgegeben werden soll, wobei die Ermittlung auf der Analyse basiert. Diese Datenmenge betrifft typischerweise Parameterwerte von Messwerten, Geräteeinstellungen, Bilder oder Informationen zur Operation. Die Datenmenge kann Daten eines einzigen Gerätes umfassen, aber auch Daten von verschiedenen Geräten.
- Abrufen von Daten dieser Datenmenge von Geräten des medizintechnischen Systems. Die Geräte werden im Rahmen der vorgenannten Kommunikation kontaktiert und die entsprechenden Daten der Datenmenge (also die Daten, die ausgegeben werden sollen) von den Geräten abgerufen.
- Darstellung der abgerufenen Daten auf einer Anzeigeeinheit. Dabei werden bevorzugt Daten von zwei oder mehr Geräten auf der Anzeigeeinheit gleichzeitig oder nacheinander dargestellt. Die abgerufenen Daten sind bevorzugt nicht patientenbezogene Daten, insbesondere Daten zu Geräteeinstellungen, sowie patientenbezogene Daten, insbesondere Parameterwerte von Messwerten, oder Bilder oder Informationen zur Operation.

Bevorzugt erfolgt eine Bereitstellung der im jeweiligen OP-Schritt (also der mittels der Analyse ermittelten aktuellen Situation) benötigten Daten oder die Veränderung von GeräteEinstellungen passend zum jeweiligen OP-Schritt und ggf. passend zu den Präferenzen des Operateurs (Benutzers).

Diese Ausführungsform hat den Vorteil, dass eine nutzerzentrierte Darstellung wesentlicher Informationen in einer einzelnen Ausgabeeinheit möglich ist. Dies erfolgt aufgrund der mittels der Kommunikation erreichten Integration der aus verschiedenen Informationsquellen und Geräten erhaltenen Informationen.

Bevorzugt erfolgt die Art der Darstellung von Daten (von Geräten des Gerätesystems) in Abhängigkeit von äußeren Umständen. Die Art der Darstellung betrifft dabei bevorzugt die Art der dargestellten Daten, die Positionierung der Darstellung im Sichtfeld und/oder besondere Hervorhebungen von Informationen. Eine besondere Hervorhebung von Informationen könnte z.B. eine Änderung der Größe oder Farbe einer dargestellten Information sein, z.B. wenn festgestellt werden sollte, dass sich diese Information in einem kritischen Wertebereich befindet.

Die Art der Darstellung von Daten kann in Abhängigkeit von dem jeweiligen Gerät erfolgen, von dem die ausgewählten Daten stammen. Bevorzugt erscheinen Daten unterschiedlicher Messgeräte an unterschiedlichen (bevorzugt individuellen) Positionen auf der Anzeigeeinheit.

Alternativ oder zusätzlich kann die Art der Darstellung von Daten in Abhängigkeit des Datentyps der ausgewählten Daten erfolgen. Bilder eines Röntgengerätes werden bevorzugt automatisch anders angezeigt als Daten zum Blutdruck.

Alternativ oder zusätzlich kann die Art der Darstellung von Daten in Abhängigkeit von einer Benutzereinstellung erfolgen. Der Benutzer kann damit vorgeben, wo und wie er welche Daten angezeigt haben möchte.

Alternativ oder zusätzlich kann die Art der Darstellung von Daten in Abhängigkeit von der Art der aktuellen Situation erfolgen. Dies lässt dem Algorithmus die Möglichkeit, automatisiert Daten in einer bestimmten Art auszugeben.

Bevorzugt ist die besagte Anzeigeeinheit eine Datenbrille. Diese ist besonders bevorzugt sowohl zur Darstellung von Augmented Reality als auch zur Darstellung von Virtual Reality ausgelegt. Dabei ist mit dem Ausdruck "Augmented Reality" eine Darstellung gemäß dem Prinzip der erweiterten Realität gemeint. Es liegt also eine Teiltransparenz des Displays vor und es erfolgt eine Vermischung realer Inhalte und virtueller Inhalte. Mit dem Ausdruck "Virtual Reality" ist eine Darstellung gemäß dem Prinzip der virtuellen Realität gemeint. Es erfolgt also eine Darstellung virtueller Inhalte vor einem undurchsichtigen Hintergrund.

Mit dem Begriff "Datenbrille" ist auch jegliche andere Art von Head-up Display umfasst. Oftmals wird jedoch der Ausdruck "Head-up Display" für Virtual-Reality-Anzeigeeinheiten verwendet. Der Begriff "Datenbrille" soll verdeutlichen, dass Virtual Reality zwar ein möglicher Anzeigemodus ist, aber auch Augmented Reality bevorzugt ist. Besonders bevorzugt ist der vorgenannte Fall, dass die Anzeigeeinheit beide Darstellungsmöglichkeiten bieten kann.

Dabei betrifft die Art der Darstellung bevorzugt die virtuelle Position der dargestellten Informationen (Daten), welche je nach der vorliegenden Abhängigkeit an einer fixen Position im Sichtfeld angezeigt werden oder mit einer fixen Position im Raum angezeigt werden (Ortsverankerung). Die dargestellten Daten können also bei einer Kopfbewegung stets gesehen werden (fixe Position im Sichtfeld) oder schweben scheinbar an festen Stellen im Operationssaal (fixe Position im Raum). Dabei kann die virtuelle Position der dargestellten Informationen bevorzugt vom Benutzer gewählt werden. Nach Präferenz des Operateurs werden diese Informationen danach entweder abhängig von der jeweilig durchgeführten Prozedur oder generisch für alle Prozeduren dargestellt.

Alternativ oder ergänzend betrifft die Art der Darstellung bevorzugt eine Darstellung im Rahmen einer Auswahl Augmented Reality oder Virtual Reality. Es kann also je nach Art der Darstellung zwischen der erweiterten Realität und der virtuellen Realität gewechselt werden.

Beispielsweise erfolgt je nach dargestelltem Datentyp eine kontextsensitive Transparenzänderung des Displays mit optionaler Einblendung von zwei- bzw. dreidimensionalen Inhalten. Röntgen- oder Mikroskopiebilder werden z.B. bevorzugt im Virtual Reality Modus dargestellt. Stromstärke der Koagulation, Vitalparameter, z.B. intraoperative elektrophysiologische Ableitungen zur Erhöhung der OP-Sicherheit, werden hingegen (bevorzugt bei laufender Operation) im Augmented Reality Modus angezeigt. Bevorzugt ist im Augmented Reality Modus auch, dass radiologische Informationen direkt mit dem Operationssitus nach Ko-Registrierung mit der realen Darstellung registriert überlagert werden.

Vorteilhaft, insbesondere bei Verwendung einer solchen Datenbrille, ist die Einbindung der künstlichen Intelligenz. Bevorzugt arbeiten Operateur und künstliche Intelligenz angelehnt an einen "Shared Control"-Ansatz gemeinsam an dem Ziel eines optimalen Operationsergebnisses. Die künstliche Intelligenz, also der Algorithmus, liegt bevorzugt in einem Rechensystem der Datenbrille, einem der Geräte des Gerätesystems, einem zusätzlichen Gerät des Gerätesystems vor oder wird von einer Computercloud als Dienst zur Verfügung gestellt.

Da der Blick des Nutzers zumindest bei einer Verwendung einer Datenbrille die Anzeigefläche vorgibt, kann die Darstellung der Informationen auf den Nutzer bezogen optimiert werden, bevorzugt bezüglich Transparenz des Hintergrunds, Schriftgrößen oder Kontraste. Dadurch werden unergonomische Körperhaltungen vermieden und weniger sowie geringere optische Fokuswechsel notwendig. Zudem ist eine standardisierte Platzierung und optimale von Informationen möglich.

Es ist auch bevorzugt, dass die Art der Darstellung von Daten von Geräten des Gerätesystems in Abhängigkeit von der Analyse erfolgt und automatisch eine Positionierung der Daten auf der Anzeigeeinheit vorgenommen wird, die für ein Gerät eine individuelle Art der Darstellung aufweist. Der Algorithmus entscheidet also aufgrund seines Trainings, eine geräteabhängige Darstellung von Daten. Dabei ist eine ergonomisch optimale Darstellung bevorzugt. Ergonomisch optimal bedeutet, dass der Benutzer zur Betrachtung der Daten keine für ihn nachteilhafte Haltung einnehmen muss. Dies führt dazu, dass die Information aus einer bestimmten Quelle immer an der gleichen, (optimalen) virtuellen Position abgerufen werden kann. Eine solche standardisierte Positionierung der Informationen ermöglicht ein fertigkeitsbasiertes Arbeiten des Operateurs.

Wohlgemerkt muss die Positionierung einer angezeigten Information nicht dort erfolgen, wo auch das entsprechende Gerät steht. Da ein Gerät platzbedingt seine Position ändern könnte (während einer Operation oder bei unterschiedlichen Operationen), wird die Information von Daten eines Gerätes bevorzugt während einer Operation und/oder bei unterschiedlichen Operationen stets an einer gleichen, vorgegebenen Position im Raum angezeigt.

Durch die Möglichkeit einer ergonomischen Anordnung der virtuellen Informationen und Eingabeoberflächen wird sowohl die Ermüdung des Operateurs, als auch dessen körperliche Belastung reduziert.

Gemäß einem bevorzugten Verfahren werden basierend auf den Handlungen des Benutzers im Zuge der Analyse Befehle des Benutzers ermittelt. Diese Befehle sind bevorzugt Sprachkommandos und/oder Steuergesten und/oder Blickzuwendung. Die datentechnische Kommunikation mit Geräten erfolgt dann in Abhängigkeit dieser Befehle, bevorzugt die Darstellung der abgerufenen Daten auf einer Anzeigeeinheit und/oder die Steuerung von Geräten des Gerätesystems. Die verschiedenen Informationsquellen (Geräte) können also benutzerinduziert abgerufen werden oder kontextsensitiv durch die künstliche Intelligenz, welche den OP-Workflow kennt, bereitgestellt werden.

Die Steuerung von verschiedenen Geräten im OP kann also über Sprachkommandos an das System (statt wie bisher an das OP-Personal) erfolgen. Es kann dabei insbesondere mittels Eye-Trackings der Blick des Benutzers als Befehl angesehen werden, z.B. eine Blickzuwendung bzw. ein Fokuswechsel des Operateurs. Alternativ oder zusätzlich kann insbesondere eine Bewegung bzw. Zuwendung des Kopfes als Befehl angesehen werden.

Bevorzugt erfolgt nach Ausführung eines Befehls eine Rückmeldung, z.B. dass die gewünschte Einstellung erreicht ist, insbesondere als Anzeige auf der Anzeigeeinheit und/oder in Form einer auditiven Rückmeldung.

Die Kopplung mit einem einheitlichen physikalischen Eingabegerät, z.B. einem Controller, zur Aufnahme von Befehlen des Benutzers ist ebenfalls bevorzugt. Es ist ebenfalls bevorzugt, dass Eingaben vom Algorithmus vorgeschlagen oder sogar selbstständig getätigt werden, jedoch jederzeit vom Benutzer mittels spezieller Kommandos wiederrufen werden können.

Gemäß einem bevorzugten Verfahren erfolgen daher basierend auf der Analyse von dem Algorithmus Vorschläge betreffend die Darstellung der abgerufenen Daten auf einer Anzeigeeinheit, deren Darstellungsart und/oder die Steuerung von Geräten des Gerätesystems. Diese Vorschläge werden bevorzugt nach einer Bestätigung durch den Benutzer im weiteren Verlauf automatisch durchgeführt. Es ist also danach möglich, dass der Algorithmus Einstellungen vorschlägt und auf Bestätigung des Operateurs auch durchführt. Gleichzeitig überprüft der Algorithmus, ob die gewünschte Einstellung für den derzeitigen OP-Schritt sinnvoll ist und fragt gegebenenfalls nach einer Bestätigung. Somit hat der Operateur die Möglichkeit diese Kontrolle durch vorgegebene Kommandos außer Kraft zu setzten und für den jeweiligen Operationsschritt untypische Einstellungen einmalig zu erlauben ("Shared Control").

Der Algorithmus ist bevorzugt in den Dimensionen Spracherkennung, Ortszuordnung und Workflow selbstlernend ausgelegt. Dadurch werden das Risiko von Kommunikations- und Einstellungsfehler reduziert.

Gemäß einem bevorzugten Verfahren antizipiert der Algorithmus nach oder im Rahmen der Analyse den jeweiligen Fortschritt der Operation und stellt die benötigten Informationen für OP-Schritte der Operation automatisch zur Verfügung. Durch die Erkennung einer Vorbereitung zu einem Operationsschritt (aktuelle Situation) kann vom Algorithmus angenommen werden, dass der betreffende Operationsschritt in nächster Zukunft folgen wird und entsprechende Vorbereitungen getroffen werden, z.B. Einstellungen von Geräten an den entsprechenden Operationsschritt automatisch angepasst werden. Zur Klassifikation von OP-Schritten im Rahmen der Operation können z.B. spezifische Kommandos, Handlungsweisen, Instrumente oder auch komplexe anatomische Informationen, insbesondere Strukturen, herangezogen werden.

Beispielsweise kann sowohl die Kommunikation im OP-Saal als auch typische Bewegungsmuster und -strukturen des Operateurs oder des OP-Personals im OP erfasst werden oder anatomische Strukturen im OP-Mikroskop. Auch Informationen von Geräten nicht operativer Fachrichtungen, z.B. zur Anästhesie werden dabei bevorzugt berücksichtigt.

Dadurch kann sichergestellt werden, dass der Operateur bei seiner chirurgischen Tätigkeit nicht durch irrelevante oder unzeitgemäße Informationen abgelenkt wird und alle wesentlichen Schritte einer Operation durchgeführt werden.

Gemäß einem bevorzugten Verfahren wird dem Benutzer ein virtuelles Eingabegerät angezeigt. In dem Fall, dass bei der Analyse erkannt wird, dass der Benutzer Eingaben an diesem virtuellen Eingabegerät durchführt, wird diese Eingabe erkannt und es erfolgt eine entsprechende Kommunikation mit einem Gerät erfolgt. Beispielsweise kann eine Einstellung gemäß Vorgabe für den jeweiligen OP-Schritt verändert werden.

Gemäß einem bevorzugten Verfahren können Eingaben des Nutzers über Sprachkommandos erfolgen. In diesem Fall ist nicht unbedingt eine explizite Anzeige notwendig, wobei ein visuell sichtbares virtuelles Eingabegerät aber durchaus als Alternative angezeigt werden kann.

Bevorzugt wird ein ausgewähltes Gerät durch einen beliebigen Klassifikator, z.B. einen Tag oder einen QR-Code auf dem Gerät beim Set-up, identifiziert. Dies geschieht bevorzugt unter Zuhilfenahme eines eindeutigen Merkmals, z.B. der ID des Tags, das in einer Datenbank hinterlegt ist. Alternativ kann eine Erfassung des Blicks oder einer Kopfzuwendung durch die Datenbrille erfolgen. Bei dieser Erfassung kann ermittelt werden, ob der Vektor eines Blicks einen getagten virtuellen Raumpunkt oder ein physikalisches Gerät trifft. In diesem Falle kann ein Auslesen der ID des Tags erfolgen und damit das Gerät gesteuert oder ausgelesen werden. Durch die Fusion mehrere Eingabemodalitäten, z.B. einer Blickzuwendung in Kombination mit einem Sprachbefehl oder einer Geste, kann eine vorteilhafte Steuerung erfolgen, z.B. Ein- und Ausschalten eines Geräts.

Bei einer bevorzugten Vorrichtung verfügt der Algorithmus über eine oder mehrere der folgenden Eigenschaften:
- Wissen erlangen über die aktuelle Situation im Operationssaal,
- Wissen erlangen über Intentionen des Benutzers im Operationssaal,
- Unterstützen des Nutzers durch Anzeige von Informationen und/oder Steuerung von Geräten, insbesondere mit dem Ziel auf eine möglichst mühelose Art und Weise eine verbesserte Leistung, einen reduzierten Arbeitsaufwand und erhöhte Sicherheit zu erreichen,
- Kommunikation einer beabsichtigten automatisierten Handlung und Abbrechen einer beabsichtigten automatisierten Handlung bei Ablehnung durch den Benutzer.

Es ist dabei besonders bevorzugt, dass der Algorithmus zwar seine Intentionen mitteilt, jedoch leicht durch den Benutzer überstimmt werden kann.

Eine bevorzugte Vorrichtung umfasst eine Datenbrille oder eine Datenschnittstelle zum Betrieb einer Datenbrille. Diese Datenschnittstelle bzw. Datenbrille ist besonders bevorzugt zur Anzeige von Daten im Rahmen von Augmented Reality als auch im Rahmen von Virtual Reality ausgelegt.

Eine Anzeige mit einer Datenbrille kann beispielsweise wie folgt realisiert werden:
Kameras liefern ein Bild des Operationssaales (Outside-in oder Inside-out), d.h. Kameras können sich außerhalb der Vorrichtung (outside-in) oder innerhalb der Vorrichtung (inside-out) befinden. Dadurch kann der Operationssaal und alle Objekte in seinem Inneren (Personen, Geräte, Werkzeuge, sonstige Gegenstände) dreidimensional erfasst werden.

Basierend auf dieser Erfassung wird eine Punktwolke aus den Daten der Kameras berechnet und abschließend erfolgt eine Ableitung der Normalvektoren von Merkmalen in ein Koordinatensystem eines dem OP entsprechenden dreidimensionalen virtuellen Raum. Nun kann eine Verknüpfung von medizinischen Daten auf Raumpunkte erfolgen, entweder auf Oberflächen oder interpoliert zwischen Oberflächen jeglicher Art, z.B. zwischen Tisch und Zimmerdecke "frei im Raum".

Besonders bevorzugt erfolgt im Rahmen der Erfindung eine Verknüpfung von medizinischen Daten auf Raumpunkte des virtuellen Operationssaales. Zur Anzeige von Daten in der Anzeigeeinheit dienen definierte Raumpunkte als Grundlage. Damit kann z.B. eine Ebene mit anzuzeigenden Daten im Speicher erzeugt werden. Danach erfolgt eine Vektortransformation der Ebene im Raum, um Daten anzuzeigen.

Bevorzugt wird der Algorithmus bei der Durchführung des Verfahrens weiter trainiert. Das Vorhandensein von Informationen aus aktuellen Guidelines im System und das Analysieren und Erlernen sich ständig ändernder Vorgehensweisen verschiedener Operateure durch einen künstlichen Intelligenzalgorithmus führt dazu, das "Systemwissen", also das "Wissen" des Algorithmus auf dem aktuellen Stand der gegenwärtigen "Best-Practices", sowie auf dem aktuellen Stand der Technik und der Wissenschaft zu halten. Bevorzugt werden die so kombinierten, ständig aktualisierten Informationen dem Operateur vorgeschlagen und durch diesen gemäß des "Shared Control" Ansatzes kontinuierlich validiert.

Durch die Erfindung kann der Zeitverlust, der z.B. durch Eingeben und Auslesen von Daten entsteht, sowie die Fehleranfälligkeit im Handeln des Operateurs und damit das OP-Risiko des Patienten reduziert werden. Weiterhin entfällt die Zeit, welche sonst für die Präparation der Daten zur optimalen Verwendung in den jeweiligen Schritten eines OP Workflows, z.B.

Scrollen/Rotieren eines 3D Objektes auf einem Bildschirm, verwenden werden müsste.

Wegen der Integration der im OP befindlichen Informationsquellen (Geräte) in einer einzigen Ausgabeeinheit ist es möglich, Informationen sowohl nutzerzentriert, also unabhängig von der tatsächlichen örtlichen Positionierung verschiedener Geräte, als auch intelligent bzw. kontextsensitiv gemäß des OP-Fortschritts anzuzeigen.

Die Reduktion des OP-Risikos für den Patienten führt, bedingt durch eine verringerte Anzahl der operationsbedingten Komplikationen, zu einem verbesserten klinischen Ergebnis und in der Konsequenz verringerte Folgekosten im Gesundheits- und Sozialsystem.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
- Figur 1: eine schematische Darstellung eines Beispiels für ein erfindungsgemäßes Gerätesystem,
- Figur 2: eine Darstellung einer bevorzugten Ausführungsform einer erfindungsgemäßen Vorrichtung,
- Figur 3: einen Ablaufplan für einen möglichen Ablauf eines erfindungsgemäßen Verfahrens.
- Figur 4: eine mögliche Darstellung gemäß dem erfindungsgemäßen Verfahren.
- Figur 5: eine weitere mögliche Darstellung gemäß dem erfindungsgemäßen Verfahren.

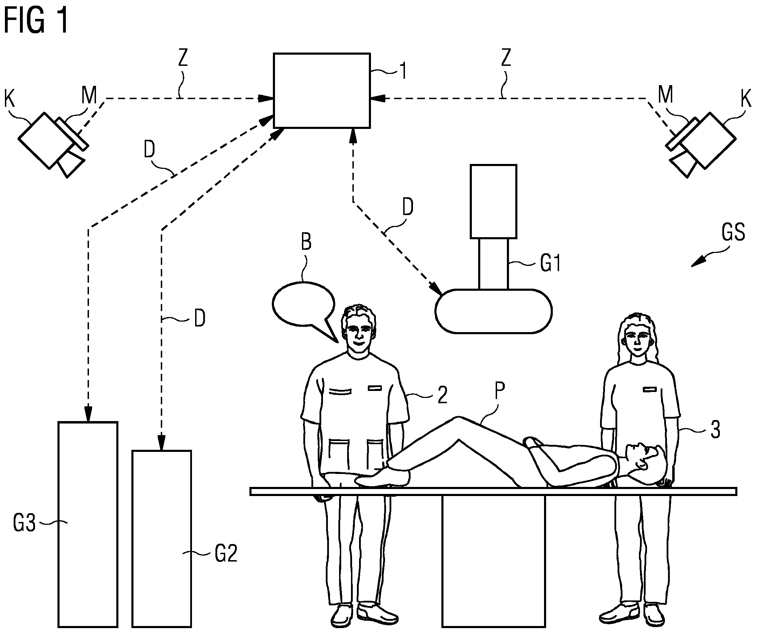

Figur 1 zeigt eine schematische Darstellung eines Beispiels für ein erfindungsgemäßes Gerätesystem GS. Die dargestellte Szene findet in einem Operationssaal statt, in dem drei unterschiedliche Geräte G1, G2, G3 für Operationen genutzt werden. Ein Patient P liegt in dem Operationssaal auf einer Liege und wird von zwei Personen operiert. Die linke Person ist in diesem Falle der Benutzer 2, die rechte Person stellt das OP-Personal 3 dar. Die drei Geräte G1, G2, G3 stehen zusammen mit zwei Kameras K und zwei Mikrofonen M in einer datentechnischen Verbindung mit einer erfindungsgemäßen Vorrichtung 1. Die Kameras K und Mikrofone M nehmen den aktuellen Zustand im Operationssaal auf (aktuelle Situation) und liefern die Zustandsdaten für das erfindungsgemäße Verfahren. Die Kameras K und Mikrofone M können zu den Geräten des Gerätesystems GS gezählt werden, sie können aber auch nicht zu dem Gerätesystem GS gezählt werden und unabhängig von diesen Zustandsdaten Z liefern. Beispielsweise könnte man festlegen, dass nur Geräte zu dem Gerätesystem GS gezählt werden, zu denen auch eine Kommunikation aufgebaut wird bzw. werden kann.

Die Bilder der Kameras K und die Aufnahmen der Mikrofone werden von der erfindungsgemäßen Vorrichtung 1 analysiert und es erfolgt datentechnischen Kommunikation mit Geräten G1, G2, G3 des Gerätesystems GS basierend auf der Analyse, was mit den auf die Geräte G1, G2, G3 deutenden Pfeilspitzen dargestellt wird. Pfeilspitzen, die auf die Vorrichtung 1 zeigen sollen Daten D anzeigen, die von den jeweiligen Geräten G1, G2, G3 (bzw. von Kameras K oder Mikrofonen M) die Vorrichtung 1 erreichen. In dem hier gezeigten Beispiel liefern einzig Kameras K und Mikrofone M Zustandsdaten Z und die Geräte G1, G2, G3 Daten D zur Anzeige, was bei anderen Ausführungsformen auch anders sein kann.

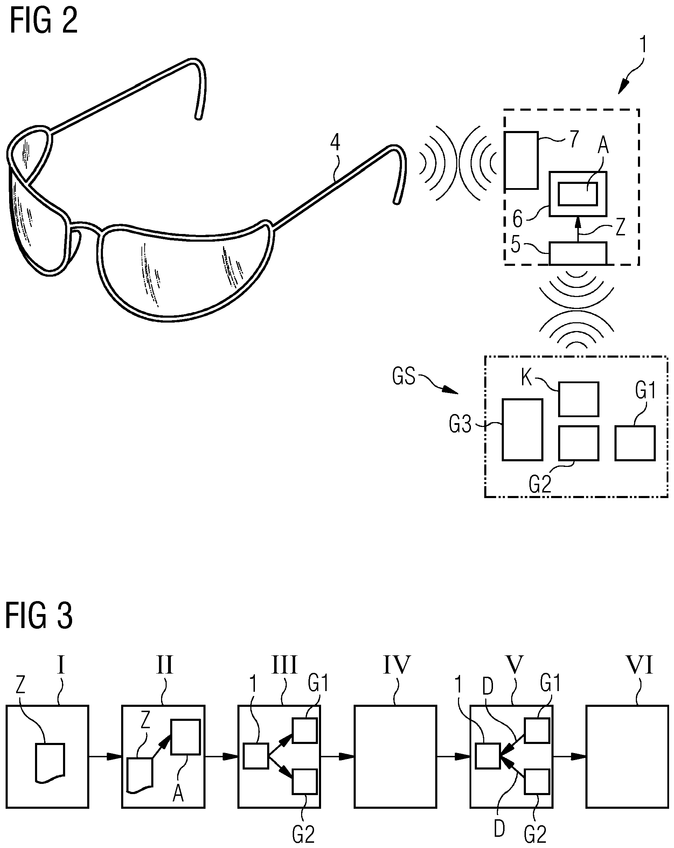

Figur 2 zeigt eine Darstellung einer bevorzugten Ausführungsform einer erfindungsgemäßen Vorrichtung 1 zur Kommunikation mit einem medizintechnischen Gerätesystem GS durch einen Benutzer 2 während einer Operation in einem Operationssaal mit einer Datenbrille 4 als Anzeigeeinheit 4. In dem hier gezeigten Fall sind Datenbrille 4 und der Rest des Systems (gestrichelter Kasten) voneinander getrennt und stehen drahtlos miteinander in einer Datenverbindung.

Die Figur könnte auch so aufgefasst werden, dass die Vorrichtung 1 eine Datenschnittstelle 7 zum Betrieb einer Datenbrille 4 umfasst. Zumindest, wenn die hier dargestellte Datenbrille 4 beliebig durch andere Modelle austauschbar wäre.

Die Vorrichtung 1 verfügt über eine Datenschnittstelle 5 zum Empfang von Zustandsdaten Z durch Geräte G1, G2, G3 des (übrigen) Gerätesystems GS, zu denen hier auch eine Kamera K gehört. Die Datenschnittstelle 5 ist hier für eine drahtlose Kommunikation ausgelegt und es erfolgt über sie auch die Kommunikation mit den Geräten G1, G2, G3 des übrigen Gerätesystems GS. Man könnte sich in diesem Beispiel auch vorstellen, dass eine Steuerung der Kamera K möglich ist, die Kamera K also anders als in Figur 1 ein Teil des Gerätesystems GS ist.

Erhält die Vorrichtung Zustandsdaten Z über eine aktuelle Situation im Operationssaal und/oder von Handlungen des Benutzers, werden diese an eine Recheneinheit 6 weitergegeben, die einen trainierten Algorithmus A basierend auf einer künstlichen Intelligenz ausgelegt zur Analyse der Zustandsdaten Z umfasst.

Die Recheneinheit 6 analysiert nun die Zustandsdaten Z (siehe dazu auch Figur 3) und es kann über die Datenschnittstelle 5 eine datentechnische Kommunikation mit Geräten G1, G2, G3 und ggf. auch der Kamera K des (übrigen) Gerätesystems GS eingeleitet werden.

Figur 3 zeigt einen Ablaufplan für einen möglichen Ablauf eines erfindungsgemäßen Verfahrens zur Kommunikation mit einem medizintechnischen Gerätesystem GS durch einen Benutzer 2 während einer Operation in einem Operationssaal. Das Verfahren umfasst die folgenden Schritte.

In Schritt I erfolgt ein Bereitstellen von Zustandsdaten Z über eine aktuelle Situation im Operationssaal und/oder von Handlungen des Benutzers 2.

In Schritt II erfolgt eine Analyse der Zustandsdaten Z mittels eines trainierten Algorithmus A basierend auf einer künstlichen Intelligenz,

In Schritt III erfolgt eine datentechnische Kommunikation mit Geräten G1, G2 des Gerätesystems GS basierend auf der Analyse. In dem hier dargestellten Fall liegt der Schwerpunkt auf dem Abrufen von Daten D dieser Geräte G1, G2.

In Schritt IV erfolgt basierend auf der Analyse eine Ermittlung derjenigen Menge von Daten D, welche während der aktuellen Situation im Operationssaal auf einem Anzeigegerät 4 (s. z.B. Fig. 2) ausgegeben werden soll.

In Schritt V erfolgt ein Abrufen von Daten D dieser Menge von Daten D von Geräten G1, G2 des medizintechnischen Systems GS.

In Schritt VI erfolgt eine Darstellung der abgerufenen Daten D auf einer Anzeigeeinheit (4), wobei z.B. Daten D von den Geräten G1, G2 auf der Anzeigeeinheit gleichzeitig oder nacheinander dargestellt werden.

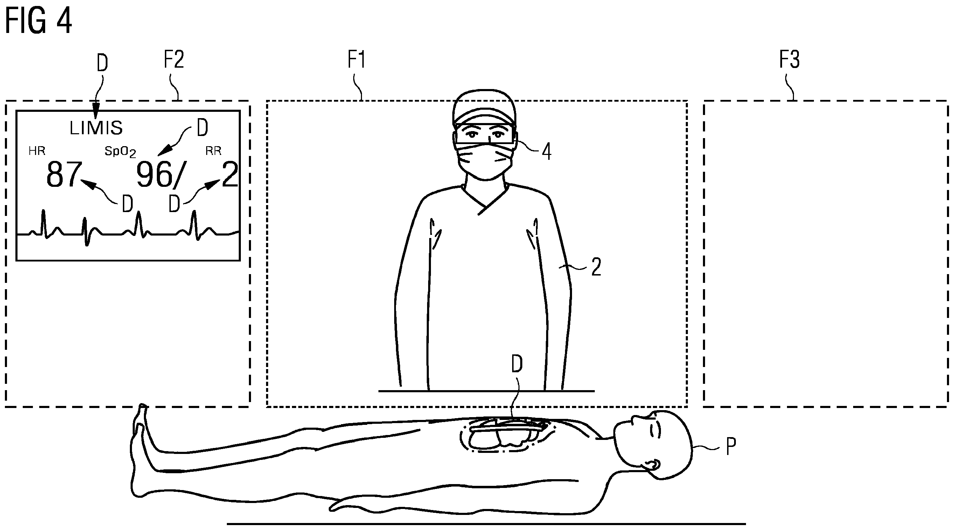

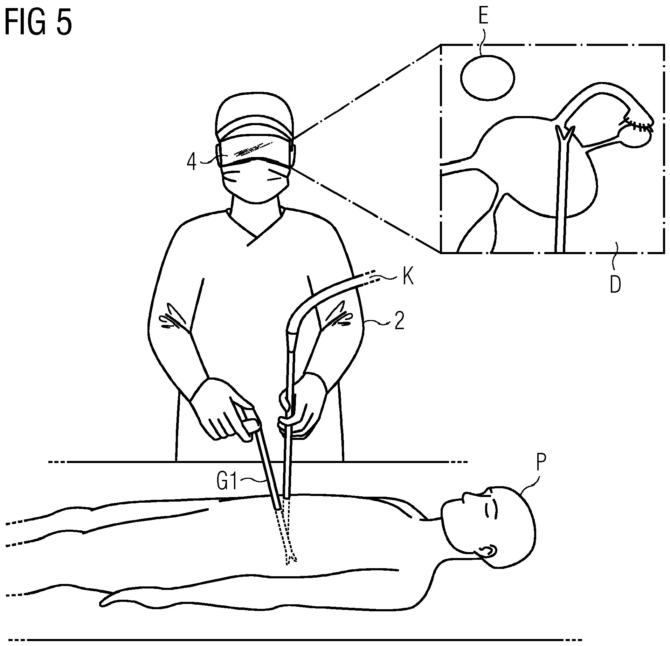

Figuren 4 und 5 zeigen mögliche Darstellungen gemäß dem erfindungsgemäßen Verfahren. Es werden dort zwei mögliche Anzeigearten einer Datenbrille 4 (s. dazu auch Figur 2) im Augmented Reality- und im Virtual Reality Modus in einem OP dargestellt.

Figur 4 zeigt dabei den Augmented Reality Modus. In diesem Modus werden Daten D verschiedener Geräte G1, G2, G3 (s. Fig. 1) sowohl an einer fixen Position im Raum angezeigt, als auch direkt mit dem Operationssitus nach Ko-Registrierung mit der realen Darstellung überlagert angezeigt. In dieser Darstellung sieht der Benutzer 2 durch ein zentrales virtuelles Fenster F1 hindurch eine virtuelle Darstellung von Daten D (Bilddaten) des Herzens des Patienten P an derjenigen Position im Raum, an der das Herz auch in der Wirklichkeit liegt. In einem linken virtuellen Fenster F2 sieht er weitere Daten D der vorgenannten Geräte G1, G2, G3, ein rechtes virtuelles Fenster F3 ist hier leer, kann aber nach einer Eingabe des Benutzers 2 (z.B. verbale Aufforderung und/oder Einsatz von Gesten und/oder Blickzuwendung auf virtuelles Eingabegerät E oder physisches Gerät G1, G2, G3) oder durch das System/künstliche Intelligenz mit Informationen gefüllt werden. Die virtuellen Fenster F1, F2, F3 können als Rahmen angezeigt werden oder nur als theoretische Aufteilungen des Sichtfeldes des Benutzers 2. Die virtuellen Fenster F1, F2, F3 können wie hier dargestellt im Raum vor dem Benutzer 2 dargestellt werden, sie können aber auch den Benutzer 2 umgeben, so dass dieser das linke virtuelle Fenster F2 und rechte Fenster F3 bei einem Blick zu seiner Linken bzw. zu seiner Rechten im Raum sieht.

Figur 5 zeigt den Virtual Reality Modus. In diesem Modus werden beispielhaft Daten D eines Gerätes G1, G2, G3 (s. Figur 1) dargestellt. In der linken oberen Ecke ist ein virtuelles Eingabegerät E in Form eines Knopfes zu erkennen, welches mit einer Geste oder einem Blick aktiviert werden kann. In der hier dargestellten Szene operiert der Benutzer 2 gerade einen Patienten P am Herzen. Er hält dabei eine Kamera K in der einen Hand, die hier als Gerät des Gerätesystems angesehen wird und sowohl Zustandsdaten Z (s. Figur 1) als auch Daten D zur Anzeige erzeugt. In seiner anderen Hand hält der Benutzer 2 ein Operationsgerät G1, dass in diesem Beispiel auch Daten D liefert und als Gerät G1 in das Gerätesystem eingebunden ist.

Wie in Figur 4 beschrieben kann die Anzeige dieses Operationsgerätes G1 durch Eingabe des Benutzers 2 (verbale Aufforderung und/oder Einsatz von Gesten und/oder Blickzuwendung auf virtuelles Eingabegerät E oder physisches Gerät G1) hervorgerufen oder durch das System/künstliche Intelligenz vorgeschlagen und nach Bestätigung des Nutzers angezeigt werden.

Es wird abschließend noch einmal darauf hingewiesen, dass es sich bei den vorhergehend detailliert beschriebenen Verfahren sowie bei der dargestellten Vorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Vorrichtung" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Kommunikation mit einem medizintechnischen Gerätesystem (GS) durch einen Benutzer (2) während einer Operation in einem Operationssaal, umfassend die Schritte:
- Bereitstellen von Zustandsdaten (Z) über eine aktuelle Situation im Operationssaal und/oder von Handlungen des Benutzers (2),
- Analyse der Zustandsdaten (Z) mittels eines trainierten Algorithmus (A) basierend auf einer künstlichen Intelligenz,
- datentechnische Kommunikation mit Geräten (G1, G2, G3) des Gerätesystems (GS) basierend auf der Analyse, wobei diese Kommunikation
a) eine Steuerung und/oder
b) ein Abrufen von Daten (D)
dieser Geräte (G1, G2, G3) umfasst.

2. Verfahren nach Anspruch 1, umfassend die Schritte:
- Ermittlung derjenigen Menge von Daten (D), welche während der aktuellen Situation im Operationssaal auf einer Anzeigeeinheit (4) ausgegeben werden soll, basierend auf der Analyse,
- Abrufen von Daten (D) dieser Menge von Daten (D) von Geräten (G1, G2, G3) des medizintechnischen Gerätesystems (GS),
- Darstellung der abgerufenen Daten (D), bevorzugt von patientenbezogenen Daten (D), auf einer Anzeigeeinheit (4),
wobei bevorzugt Daten (D) von zwei oder mehr Geräten (G1, G2, G3) auf der Anzeigeeinheit (4) gleichzeitig oder nacheinander dargestellt werden.

3. Verfahren nach Anspruch 2, wobei die Art der Darstellung von Daten (D) in Abhängigkeit von
- dem jeweiligen Gerät (G1, G2, G3) von dem die ausgewählten Daten (D) stammen und/oder
- des Datentyps der ausgewählten Daten (D) und/oder
- einer Benutzereinstellung und/oder
- von der Art der aktuellen Situation
erfolgt,
wobei die Art der Darstellung bevorzugt die Art der dargestellten Daten (D), die Positionierung der Darstellung im Sichtfeld und/oder besondere Hervorhebungen von Informationen betrifft.

4. Verfahren nach Anspruch 2 oder 3, wobei die Anzeigeeinheit (4) eine Datenbrille (4) ist, die bevorzugt sowohl zur Darstellung von Augmented Reality als auch zur Darstellung von Virtual Reality ausgelegt ist,
wobei bevorzugt die Art der Darstellung
- die virtuelle Position der dargestellten Daten (D) betrifft, welche je nach der vorliegenden Abhängigkeit an einer fixen Position im Sichtfeld angezeigt werden oder mit einer fixen Position im Raum angezeigt werden,
und/oder
- eine Darstellung im Rahmen einer Auswahl Augmented Reality oder Virtual Reality betrifft.

5. Verfahren nach einem der Ansprüche 2 bis 4, wobei die Art der Darstellung von Daten (D) von Geräten (G1, G2, G3) des Gerätesystems (GS) in Abhängigkeit von der Analyse erfolgt und automatisch eine Positionierung der Daten (D) auf der Anzeigeeinheit (4) vorgenommen wird, die für ein Gerät (G1, G2, G3) eine individuelle Art der Darstellung aufweist, bevorzugt eine ergonomisch optimale Darstellung.

6. Verfahren nach einem der vorangehenden Ansprüche, wobei basierend auf den Handlungen des Benutzers (2) im Zuge der Analyse Befehle (B) des Benutzers (2) ermittelt werden und die datentechnische Kommunikation mit Geräten (G1, G2, G3) in Abhängigkeit dieser Befehle (B) erfolgt,
bevorzugt die Darstellung der abgerufenen Daten (D) auf einer Anzeigeeinheit (4) und/oder die Steuerung von Geräten (G1, G2, G3) des Gerätesystems (GS),
wobei bevorzugt nach Ausführung eines Befehls (B) eine Rückmeldung erfolgt.

7. Verfahren nach einem der vorangehenden Ansprüche, wobei basierend auf der Analyse von dem Algorithmus (A) ein Vorschlag betreffend die Darstellung der abgerufenen Daten (D) auf einer Anzeigeeinheit (4), deren Art der Darstellung und/oder die Steuerung von Geräten (G1, G2, G3) des Gerätesystems (GS) erfolgt, welcher bevorzugt nach einer Bestätigung durch den Benutzer (2) im weiteren Verlauf automatisch durchgeführt wird.

8. Verfahren nach einem der vorangehenden Ansprüche, wobei der Algorithmus (A) nach oder im Rahmen der Analyse den jeweiligen Fortschritt der Operation antizipiert und die benötigten Informationen für OP-Schritte der Operation automatisch zur Verfügung stellt, wobei zur Klassifikation von OP-Schritten im Rahmen der Operation spezifische Kommandos, Handlungsweisen, Instrumente oder auch komplexe anatomische Informationen herangezogen werden.

9. Verfahren nach einem der vorangehenden Ansprüche, wobei dem Benutzer (2) ein virtuelles Eingabegerät (E) angezeigt wird und/oder eine Eingabe des Nutzers über Sprachkommandos erfolgt und in dem Falle dass bei der Analyse erkannt wird, dass der Benutzer (2) Eingaben an diesem virtuellen Eingabegerät (E) und/oder über Sprachkommandos durchführt, diese Eingabe erkannt wird und eine entsprechende Kommunikation mit einem Gerät (G1, G2, G3) erfolgt.

10. Vorrichtung zur Kommunikation mit einem medizintechnischen Gerätesystem (GS) durch einen Benutzer (2) während einer Operation in einem Operationssaal umfassend:
- eine Datenschnittstelle (5) zum Empfang von Zustandsdaten (Z) über eine aktuelle Situation im Operationssaal und/oder von Handlungen des Benutzers (2),
- eine Recheneinheit (6) mit einem trainierten Algorithmus (A) basierend auf einer künstlichen Intelligenz ausgelegt zur Analyse der Zustandsdaten (Z),
- eine Datenschnittstelle (5) ausgelegt zur datentechnischen Kommunikation mit Geräten (G1, G2, G3) des Gerätesystems (GS) basierend auf der Analyse, wobei diese Kommunikation
a) eine Steuerung und/oder
b) ein Abrufen von Daten (D)
dieser Geräte (G1, G2, G3) umfasst.

11. Vorrichtung nach Anspruch 10, wobei der Algorithmus (A) über eine oder mehrere der folgenden Eigenschaften verfügt:
- Wissen erlangen über die aktuelle Situation im Operationssaal,
- Wissen erlangen über Intentionen des Benutzers (2) im Operationssaal,
- Unterstützen des Benutzers (2) durch Anzeige von Informationen und/oder Steuerung von Geräten (G1, G2, G3),
- Kommunikation einer beabsichtigten automatisierten Handlung und Abbrechen einer beabsichtigten automatisierten Handlung bei Ablehnung durch den Benutzer (2).

12. Vorrichtung nach Anspruch 10 oder 11, umfassend eine Datenbrille (4) oder eine Datenschnittstelle (7) zum Betrieb einer Datenbrille (4), wobei die Vorrichtung bevorzugt zur Anzeige von Daten (D) im Rahmen von Augmented Reality als auch im Rahmen von Virtual Reality ausgelegt ist.

13. Medizintechnisches Gerätesystem umfassend eine Vorrichtung (1) nach einem der Ansprüche 10 bis 12 und/oder ausgelegt zur Durchführung eines erfindungsgemäßen Verfahrens nach einem der Ansprüche 1 bis 9.

14. Computerprogrammprodukt mit einem Computerprogramm, welches direkt in eine Speichereinrichtung eines Rechensystems ladbar ist, mit Programmabschnitten, um alle Schritte des Verfahrens nach einem der Ansprüche 1 bis 9 auszuführen, wenn das Computerprogramm in dem Rechensystem ausgeführt wird.

15. Computerlesbares Medium, auf welchem von einem Rechensystem einlesbare und ausführbare Programmabschnitte gespeichert sind, um alle Schritte des Verfahrens nach einem der Ansprüche 1 bis 9 auszuführen, wenn die Programmabschnitte von dem Rechensystem ausgeführt werden.

## Geänderte Patentansprüche

### Geänderte Patentansprüche gemäss Regel 137(2) EPÜ.

1. Verfahren zur Kommunikation mit einem medizintechnischen Gerätesystem (GS) durch einen Benutzer (2) während einer Operation in einem Operationssaal, umfassend die Schritte:
- Bereitstellen von Zustandsdaten (Z) über eine aktuelle Situation im Operationssaal und/oder von Handlungen des Benutzers (2),
- Analyse der Zustandsdaten (Z) mittels eines trainierten Algorithmus (A) basierend auf einer künstlichen Intelligenz, wobei die aktuell vorliegende Situation mittels der Analyse ermittelt wird,
- datentechnische Kommunikation mit Geräten (G1, G2, G3) des Gerätesystems (GS) basierend auf der Analyse, wobei
- basierend auf der Analyse eine Ermittlung derjenigen Menge von Daten (D) erfolgt, welche während der aktuellen Situation im Operationssaal auf einer Anzeigeeinheit (4) ausgegeben werden soll, und wobei diese Kommunikation ein Abrufen von Daten (D) dieser Menge von Daten (D) dieser Geräte (G1, G2, G3) umfasst,
- Darstellung der abgerufenen Daten (D) auf einer Anzeigeeinheit (4).

2. Verfahren nach Anspruch 1, wobei die datentechnische Kommunikation zusätzlich eine Steuerung von Geräten (G1, G2, G3) des Gerätesystems (GS) basierend auf der Analyse umfasst.

3. Verfahren nach Anspruch 2, wobei die Art der Darstellung von Daten (D) in Abhängigkeit von
- dem jeweiligen Gerät (G1, G2, G3) von dem die ausgewählten Daten (D) stammen und/oder
- des Datentyps der ausgewählten Daten (D) und/oder
- einer Benutzereinstellung und/oder
- von der Art der aktuellen Situation
erfolgt,
wobei die Art der Darstellung bevorzugt die Art der dargestellten Daten (D), die Positionierung der Darstellung im Sichtfeld und/oder besondere Hervorhebungen von Informationen betrifft.

4. Verfahren nach Anspruch 2 oder 3, wobei die Anzeigeeinheit (4) eine Datenbrille (4) ist, die bevorzugt sowohl zur Darstellung von Augmented Reality als auch zur Darstellung von Virtual Reality ausgelegt ist,
wobei bevorzugt die Art der Darstellung
- die virtuelle Position der dargestellten Daten (D) betrifft, welche je nach der vorliegenden Abhängigkeit an einer fixen Position im Sichtfeld angezeigt werden oder mit einer fixen Position im Raum angezeigt werden, und/oder
- eine Darstellung im Rahmen einer Auswahl Augmented Reality oder Virtual Reality betrifft.

5. Verfahren nach einem der Ansprüche 2 bis 4, wobei die Art der Darstellung von Daten (D) von Geräten (G1, G2, G3) des Gerätesystems (GS) in Abhängigkeit von der Analyse erfolgt und automatisch eine Positionierung der Daten (D) auf der Anzeigeeinheit (4) vorgenommen wird, die für ein Gerät (G1, G2, G3) eine individuelle Art der Darstellung aufweist, bevorzugt eine ergonomisch optimale Darstellung.

6. Verfahren nach einem der vorangehenden Ansprüche, wobei basierend auf den Handlungen des Benutzers (2) im Zuge der Analyse Befehle (B) des Benutzers (2) ermittelt werden und die datentechnische Kommunikation mit Geräten (G1, G2, G3) in Abhängigkeit dieser Befehle (B) erfolgt,
bevorzugt die Darstellung der abgerufenen Daten (D) auf einer Anzeigeeinheit (4) und/oder die Steuerung von Geräten (G1, G2, G3) des Gerätesystems (GS),
wobei bevorzugt nach Ausführung eines Befehls (B) eine Rückmeldung erfolgt.

7. Verfahren nach einem der vorangehenden Ansprüche, wobei basierend auf der Analyse von dem Algorithmus (A) ein Vorschlag betreffend die Darstellung der abgerufenen Daten (D) auf einer Anzeigeeinheit (4), deren Art der Darstellung und/oder die Steuerung von Geräten (G1, G2, G3) des Gerätesystems (GS) erfolgt, welcher bevorzugt nach einer Bestätigung durch den Benutzer (2) im weiteren Verlauf automatisch durchgeführt wird.

8. Verfahren nach einem der vorangehenden Ansprüche, wobei der Algorithmus (A) nach oder im Rahmen der Analyse den jeweiligen Fortschritt der Operation antizipiert und die benötigten Informationen für OP-Schritte der Operation automatisch zur Verfügung stellt, wobei zur Klassifikation von OP-Schritten im Rahmen der Operation spezifische Kommandos, Handlungsweisen, Instrumente oder auch komplexe anatomische Informationen herangezogen werden.

9. Verfahren nach einem der vorangehenden Ansprüche, wobei dem Benutzer (2) ein virtuelles Eingabegerät (E) angezeigt wird und/oder eine Eingabe des Nutzers über Sprachkommandos erfolgt und in dem Falle dass bei der Analyse erkannt wird, dass der Benutzer (2) Eingaben an diesem virtuellen Eingabegerät (E) und/oder über Sprachkommandos durchführt, diese Eingabe erkannt wird und eine entsprechende Kommunikation mit einem Gerät (G1, G2, G3) erfolgt.

10. Vorrichtung zur Kommunikation mit einem medizintechnischen Gerätesystem (GS) durch einen Benutzer (2) während einer Operation in einem Operationssaal umfassend:
- eine Datenschnittstelle (5) zum Empfang von Zustandsdaten (Z) über eine aktuelle Situation im Operationssaal und/oder von Handlungen des Benutzers (2),
- eine Recheneinheit (6) mit einem trainierten Algorithmus (A) basierend auf einer künstlichen Intelligenz ausgelegt zur Analyse der Zustandsdaten (Z), wobei die aktuell vorliegende Situation mittels der Analyse ermittelt wird,
- eine Datenschnittstelle (5) ausgelegt zur datentechnischen Kommunikation mit Geräten (G1, G2, G3) des Gerätesystems (GS) basierend auf der Analyse,
wobei die Vorrichtung dazu ausgelegt ist, basierend auf der Analyse eine Ermittlung derjenigen Menge von Daten (D) durchzuführen, welche während der aktuellen Situation im Operationssaal auf einer Anzeigeeinheit (4) ausgegeben werden soll, und wobei diese Kommunikation ein Abrufen von Daten (D) dieser Menge von Daten (D) dieser Geräte (G1, G2, G3) umfasst,
- eine Anzeigeeinheit (4) ausgelegt zur Darstellung der abgerufenen Daten (D).

11. Vorrichtung nach Anspruch 10, wobei der Algorithmus (A) über eine oder mehrere der folgenden Eigenschaften verfügt:
- Wissen erlangen über die aktuelle Situation im Operationssaal,
- Wissen erlangen über Intentionen des Benutzers (2) im Operationssaal,
- Unterstützen des Benutzers (2) durch Anzeige von Informationen und/oder Steuerung von Geräten (G1, G2, G3),
- Kommunikation einer beabsichtigten automatisierten Handlung und Abbrechen einer beabsichtigten automatisierten Handlung bei Ablehnung durch den Benutzer (2).

12. Vorrichtung nach Anspruch 10 oder 11, umfassend eine Datenbrille (4) oder eine Datenschnittstelle (7) zum Betrieb einer Datenbrille (4), wobei die Vorrichtung bevorzugt zur Anzeige von Daten (D) im Rahmen von Augmented Reality als auch im Rahmen von Virtual Reality ausgelegt ist.

13. Medizintechnisches Gerätesystem umfassend eine Vorrichtung (1) nach einem der Ansprüche 10 bis 12 und/oder ausgelegt zur Durchführung eines erfindungsgemäßen Verfahrens nach einem der Ansprüche 1 bis 9.

14. Computerprogrammprodukt mit einem Computerprogramm, welches direkt in eine Speichereinrichtung eines Rechensystems ladbar ist, mit Programmabschnitten, um alle Schritte des Verfahrens nach einem der Ansprüche 1 bis 9 auszuführen, wenn das Computerprogramm in dem Rechensystem ausgeführt wird.

15. Computerlesbares Medium, auf welchem von einem Rechensystem einlesbare und ausführbare Programmabschnitte gespeichert sind, um alle Schritte des Verfahrens nach einem der Ansprüche 1 bis 9 auszuführen, wenn die Programmabschnitte von dem Rechensystem ausgeführt werden.
